# EUROPÄISCHE PATENTSCHRIFT

(11) **EP 1 419 761 B1**
(45) Veröffentlichungstag und Bekanntmachung des Hinweises auf die Patenterteilung: **19.10.2005**
(21) Anmeldenummer: 03026137.4
(22) Anmeldetag: 13.11.2003
(51) Int. Cl.: A61K 7/48, A61K 7/02

(54) **Mischung enthaltend einen Komplex aus alpha-Cyclodextrin und Vitamin F**
Mixture comprising a complex of cyclodextrin and vitamin F
Mélange contenant un complexe de cyclodextrine et de la vitamine F

(30) Priorität: 14.11.2002 DE 10253042
(43) Veröffentlichungstag der Anmeldung: 19.05.2004
(73) Patentinhaber: Wacker-Chemie GmbH, 81737 München (DE)
(72) Erfinder: Regiert, Marlies, 80538 München (DE); Kupka, Michaela, 84489 Burghausen (DE)
(74) Vertreter: Potten, Holger

(56) Entgegenhaltungen:
- EP-A- 0 470 452
- EP-A- 1 041 136
- WO-A-02/38123
- WO-A-97/09039
- WO-A-97/36972
- CH-A- 689 373
- US-A- 4 732 759
- US-A- 4 775 749
- DATABASE WPI Week 199427 Derwent Publications Ltd., London, GB; AN 1994-220438 XP002248894 & JP 06 153860 A (NISSEI KOSAN KK), 3. Juni 1994 (1994-06-03)
- SZENTE ET AL.: "Fatty Acid-Cyclodextrin Complexes: Properties and Applications" JOURNAL OF INCLUSION PHENOMENA AND MOLECULAR RECOGNITION IN CHEMISTRY, Bd. 16, 1993, Seiten 339-354, XP000675556
- DATABASE WPI Week 199541 Derwent Publications Ltd., London, GB; AN 1995-317436 XP002036274 & JP 07 215911 A (ENSUIKO SUGAR REFINING CO. LTD.), 15. August 1995 (1995-08-15)
- LANDAUS: "International Dictionnary of Medicine and Biology" 1986 , JOHN WILEY & SONS , USA VOL. III XP002264554 * Seite 3159, "vitamin F" *

## Beschreibung

Die Erfindung betrifft eine Mischung enthaltend einen Komplex aus α-Cyclodextrin und Vitamin F.

Cyclodextrine sind cyclische Oligosaccharide, die aus 6,7 oder 8 α(1-4)-verknüpften Anhydroglukoseeinheiten aufgebaut sind. Die durch enzymatische Stärkekonversion hergestellten α-, β- oder γ-Cyclodextrine unterscheiden sich in dem Durchmesser ihrer hydrophoben Kavität und eignen sich generell zum Einschluss zahlreicher lipophiler Substanzen.

Vitamin F, besteht im wesentlichen aus einer Mischung aus essentiellen Fettsäuren (Essential Fatty Acids - EFA), insbesondere Omega-6-polyungesättigten Fettsäuren. Im Sinne der vorliegenden Erfindung wird unter Vitamin F eine ungesättigte Fettsäure mit einer Kettenlänge größer bzw. gleich 18 Kohlenstoffatomen, die mindestens zwei Doppelbindungen aufweist, vorzugsweise EFA, insbesondere Omega-6-polyungesättigte Fettsäure, verstanden. EFA kann der Körper nicht selbst in der benötigten Menge herstellen, eine ausreichende Versorgung ist jedoch für natürliche Funktionen des Körpers essentiell. Der in vorliegender Anmeldung verwendete Ausdruck "essentielle Fettsäuren" entspricht Vitamin F. Bei den Omega-6-polyungesättigten Fettsäuren handelt es sich besonders bevorzugt um Linolsäure und ihre Isomere, um Linolensäure und ihre Isomeren sowie anderen sehr oxidationsempfindliche Säuren. Vitamin F entfaltet, wie sich gezeigt hat, bestimmte Eigenschaften, die u.a. zur Verbesserung des Aussehens der Haut besonders erwünscht sind.

Bei Untersuchungen konnte insbesondere gezeigt werden, dass Kombinationen aus mindestens einer essentiellen Fettsäure oder Mischungen davon und insbesondere von Vitamin F eine bemerkenswerte Oxidationsbeständigkeit aufweisen, die sich dann nicht zeigt, wen man essentiellen Fettsäuren und insbesondere Vitamin F nicht in Form der erfindungsgemäßen Wirkstoffkombination verwendet.

Die topische Anwendung von Vitaminen in der Kosmetik wurde bereits von einer Reihe von Autoren vorgeschlagen. Als besonders empfohlene Vitamine kann man nennen Vitamin A, Vitamin B, die Vitamine B₂, und B₆, Vitamin E, Vitamin H bzw. deren Cyclodextrinkomplexe sowie bestimmte geeignete Vitaminmischungen wie eine Mischung aus Vitamin A, Vitamin E und Vitamin D₃, welche Kombination einen synergistischen Effekt aufweist.

Das Vitamin, das das besondere Interesse der Formulierer von Kosmetikprodukten gefunden hat, ist das Vitamin F. Es übt eine günstige Wirkung auf trockene und rauhe Haut sowie auf die Haut aus, die bestimmte Reizanzeichen zeigt. Die Anwendung des Vitamins F in der Kosmetik wurde stets von dem besonders akuten Problem beeinträchtigt, das im wesentlichen in der großen Instabilität dieses Materials gegen die Oxidation durch den Sauerstoff der Atmosphäre zu sehen ist. Es hat sich insbesondere gezeigt, dass sich sehr schnell nach Lagerung bzw. der ersten Benutzung Zersetzungsprodukte mit ranzigem Geruch bilden, die die weitere Anwendung von kosmetischen Präparaten auf der Grundlage dieses Vitamins verhindern. Da das Vitamin F im wesentlichen aus Linolsäure und ihren Isomeren und insbesondere aus Linolensäure und ihren Isomeren und anderen sehr oxidationsempfindlichen Säuren besteht, hat man vorzugsweise in der Kosmetik sowie in Lebensmitteln die entsprechenden Alkohole oder die Ester z.B. die Triglyceride dieser Säure verwendet, die stabiler sind, wobei in diesem Fall ein erheblicher Aktivitätsverlust hingenommen werden muss. Möglichkeiten zur Stabilisierung der essentiellen Fettsäuren sind somit von außerordentlichem Interesse.

Der in vorliegender Anmeldung verwendete Ausdruck "essentielle Fettsäuren" umfasst besonders bevorzugt ungesättigte Fettsäuren, die mindestens zwei Doppelbindungen aufweisen, wie:
Linolsäure oder 9,12-Octadecadiensäure der Formel

   CH₃ (CH₂)₄ CH = CH CH₂ CH = CH (CH₂)₇ COOH

   und deren Stereoisomere, insbesondere das z-9, z-12-Isomer, sowie deren Stellungsisomere oder konjugierte Linolsäuren, d.h.:
9,11-Octadecadiensäure der Formel

   CH₃ (CH₂)₅ CH = CH CH = CH (CH₂)₇ COOH

   und deren Stereoisomere
10,12-Octadecadiensäure der Formel

   CH₃ (CH₂)₄ CH = CH CH = CH (CH₂)₈ COOH

   und deren Stereoisomere;
α-Linolensäure oder 9,12,15-Octadecatriensäure der Formel

   CH₃ (CH₂ CH = CH)₃ CH₂ (CH₂)₆ COOH

   und deren Stereoisomere und insbesondere das z-9, z-12, z-15-Isomer;
γ-Linolensäure oder 6,9,12-Octadecatriensäure der Formel

   CH₃ (CH₂ CH = CH)₃(CH₂)₇ COOH

   und deren Stereoisomere; und
Arachidonsäure oder 5,8,11,14-Eicosatetraensäure der Formel

   CH₃ (CH₂)₄ (CH = CH CH₂)₄ CH₂ CH₂ COOH

Wichtige physiologische Funktionen von Vitamin F u.a. wegen des hohen Anteils von Linolsäure sind der Aufbau der Arachidonsäure, einem Bestandteil von Phospholipiden (Zellmembrane) und Prostaglandinen (Autocoide), die Einfluss auf den Serumcholesterin-Spiegel und den Blutdruck nehmen. Ein Mangel an Linolsäure führt bei Kindern zu Wachstumsstörungen, erhöhter Infektanfälligkeit, Hautveränderungen (Ekzemen) und zu einer gestörten Funktion der Kapillaren. Vitamin F wird bei Wundheilungsstörungen eingesetzt. Die bereits in geringer Konzentration festzustellenden hautpflegenden Eigenschaften machen es zu einer äußerst wertvollen Substanz. Im Bereich der Kosmetik besteht daher ein großes Interesse am verstärkten Einsatz von Vitamin F besonders in dermatologischen Formulierungen. Die topische Applikation von Vitamin F stabilisiert den Haushalt der Haut an essentiellen Fettsäuren. Eine Unterversorgung mit Vitamin F führt zu einer Schädigung speziell der Zellmembrane der Haut (Phospholipidstruktur) als auch in den Hautlipiden (Ceramid Struktur) zu verstärkter Falten- und Hornbildung ("Photoaging") und zu einem Elastizitätsverlust der Haut. Die Hautlipide mit einem hohen Anteil an mehrfachungesättigten Fettsäuren, wie der Linolsäure, zeigen eine höhere Packungsdichte, welche offenbar die Barrierefunktion gegenüber Mikroorganismen stärkt sowie einen verminderten trans-epidermalen Wasserverlust. Dies konnte an Versuchen auf menschlicher wie tierischer Haut bei topischer Anwendung bewiesen werden. Zudem werden aus der Linolsäure Hautlipide synthetisiert.
Das Hauptproblem für die breitere Anwendung von Vitamin F besteht in seiner Empfindlichkeit gegen Oxidation, insbesondere unter Lichteinwirkung.
An der konjugiert ungesättigten Kohlenwasserstoffkette des Fettsäuremoleküls findet eine Autoxidation statt, die zur Bildung zahlreicher Zersetzungsprodukte, zu Isomerisierungen und Polymerisationen führt. Beim Ranzigwerden von Fetten kann beispielsweise die Linolsäure, eine der essentiellen Fettsäuren in eine isomere Fettsäure mit konjugierter Doppelbindung über gehen, die keinen Vitamin-Charakter mehr besitzt und Mangelschäden sogar verstärken kann. Aus der ursprünglich öligen, dünnflüssigen Substanz entsteht dabei eine zäh-flüssige Masse. Der Geruch wird als intensiv ranzig wahrgenommen. Durch intermediär gebildete Peroxide erhöht sich das toxische Potential der Formulierungen, der kosmetisch wie ernährungsphysiologisch erwünschte Effekt des intakten Vitamin F wird verringert.

Wie bereits angegeben, besteht Vitamin F im wesentlichen aus Linolsäure und deren Isomeren, wobei insbesondere bevorzugt das 9,12-Isomer in einer Menge enthalten ist, die zwischen etwa 40 und 70% liegt, wobei der Gesamtanteil der Linolsäure und deren Isomeren etwa 80 bis 90% des Materials bildet, während der Rest des Materials im wesentlichen aus einer Mischung von anderen essentiellen Fettsäuren besteht.

In CH 646049 wird die Herstellung von oxidationsbeständigen kosmetischen Zubereitungen, die Vitamin F bzw. essentielle Fettsäuren enthalten mittels eines pflanzlichen Öles nämlich Jojoba-Öl, beschrieben.

In Biol. Food Chem. Aspects, Contrib. Lipidforum/Sik Symp., Meeting Date 1985, 165-70 wird die Stabilisierung von Lipiden über den molekularen Einschluss von Linolsäure mit Cyclodextrinen mit Casein als Antioxidant in Dispersion, beschrieben.

Aus Biochem. J. (1995) 308, 151-154 (Print in Great Britain) ist der Einsatz von "löslichen Lipiden" für biochemische Prozesse mittels Linolsäure-CD-Komplexe in wässriger Lösung, bekannt

In der japanischen Patentschrift 60181014A ist die Herstellung von Badeprodukt -Zusätzen mit Vitaminen u.a. Vitamin F mittels Cyclodextrinen u. Diastase beschrieben.

Aus US-A 4,775,749 sind Komplexe aus α-Cyclodextrin und Eicosapentensäure sowie deren kosmetische Anwendung bekannt.

Aufgabe der vorliegenden Erfindung ist es, eine Mischung enthaltend Vitamin F, zur Verfügung zu stellen, die gegenüber oxidativer Zersetzung stabilisiert ist.

Die Aufgabe wird gelöst durch eine Mischung gemäß Anspruch 1. Die Erfindung betrifft ferner eine Zubereitung gemäß Anspruch 2.

Die erfindungsgemäße kosmetische oder dermatologische Zubereitung ist oxidationsbeständig und eignet sich bevorzugt zur Pflege und zum Schutz der Haut, insbesondere der empfindlichen und der trockenen Haut. Insbesondere behebt sie die mit einer Hautalterung verbundenen Schäden und dient der Prophylaxe dauerhaft und ohne Risiko von Nebenwirkungen, indem sie die Nachteile des Standes der Technik vermeiden.

Ein Komplex aus Vitamin F und Cyclodextrin (CD) kann dabei in an sich bekannter Weise aus z.B. Lösung oder mit Pastenmethode hergestellt werden. Als vorteilhaft hat sich die Herstellung aus konzentrierten, wässrigen CD-Lösungen erwiesen. Die CD-Konzentration der wässrigen Lösungen liegt vorzugsweise zwischen 5-50 Gewichts-%, bevorzugt ist eine CD-Konzentration von 20-50%.

Das Gewichts-Verhältnis von Vit- F zu CD liegt zwischen 1 : 4 und 1 : 40, bevorzugt zwischen 1: 15 und 1 : 20. Die Ansätze werden je nach Konsistenz intensiv gerührt oder geknetet.

Die Reaktionstemperatur liegt üblicherweise bei 20 - 85°C. Bevorzugt wird bei 20 - 75 °C, besonders bevorzugt bei 50 - 70 °C, gearbeitet. Die Reaktionsdauer hängt von der Temperatur ab und liegt vorzugsweise zwischen einer Stunde und einigen Tagen. Bevorzugt ist eine Reaktionszeit von 30 bis 80 Stunden.

Die Komplexierung erfolgt in der Regel unter Normaldruck. Bevorzugt findet die Komplexierung unter Schutzgasatmosphäre (Stickstoff oder Argon) statt.

Die wenig wasserlöslichen Komplexe aus Vit. F und CD können direkt verwendet werden. Sie können aber auch durch Filtration, Zentrifugation, Trocknung, Mahlen, Sieben, Sichten, Granulieren, Tablettieren entsprechend isoliert und aufbereitet werden.

Die kosmetischen und dermatologische Formulierungen, die Wirkstoffkombination von Cyclodextrin mit Vit. F enthalten, liegen in verschiedenen Formen vor z.B. als Emulsionen vom Typ Wasser-in-Öl (W/O) oder Öl-in-Wasser (O/W), Zubereitungen dieser Art sind Milchpräparate, Lotionen, Cremes oder Salben sowie Gels, Puder, Masken, Packungen, Sprays, Aerosole oder Stifte die entweder zum Färben der Lippen oder zur Behandlung von aufgesprungenen Lippen dienen, oder Schminkprodukten für die Augen oder der Gesichtsschminke.

Eine erfindungsgemäße Mischung kann auch auf Textilien bzw. Non Wovens aufgebracht werden und damit über dieses Medium der Haut zur Verfügung gestellt werden.

Eine erfindungsgemäße kosmetische und dermatologische Zubereitung enthält lineare wie cyclische Silikonöle z.B. Dimethicone sowie Cyclomethicone
feuchthaltende Mittel, also Substanzen, welche die Haut vor dem Austrocknen schützen, wie Propylenglycol, Mg-Sulfat, Glycerin,
Substanzen, welche die Haut pflegen, wie Cetyl Alkohol, Paraffinium liquide, Petrolatum, Caprylic/Capric Tiglyceride, Mineralöle, Stearinsäure, Bienen-, Candillawachs, Isopropyl-und Myristyl Myristate, Octyldodecanol, Octyldodecyllanolate, Polyethylenglycol (PEG)-22/Dodecyl Glycol Copolymer, Hydrolisiertes Weizenkeimprotein, natürliche Öle z.B. Sonnenblumen-, Soja-, Mandel-, Palm-, Kokos- und Olivenöl
Gel-Bildner, wie Salze des Carbopols, Polymethacrylate, Polysacharide
O/W sowie W/O-Emulgatoren, z. B.: Polysorbate 20, PEG-40 Stearate, PEG Hydrogenated Castor Oil, Aluminium-, Octyl- oder Glyceryl Stearate, Lecithin,
Konservierungsmittel wie Urea, Chlorhexidine Digluconate, Phenoxyethanol, Natriumbenzoat, Sorbinsäure, Methyl-, Ethyl-, Butylparabene, BHT, BHA,
Bakterizide, Antioxidantien wie. Vitamin C und entsprechende Derivate, alpha-Hydroxysäuren und entsprechende Derivate, Vitamin E und entsprechende Derivate, Sonnenschutzfilter wie UVA-und UVB-Filter, z.B. Oxyde 4-Methylbenzylidene Camphor, DEA-methoxycinnamate, Benzophenone-4, Octyldimethyl PABA, anorganische Pigmente wie Oxyde von Titanium, Eisen, Zink, Selbstbräunungsmittel wie: Dihydroxyacetone, Zusatzstoffe- und Hilfsstoffe beispielsweise Insektrepellentien, Konsistenzgeber, Füllstoffe, Elektrolyte, Vitamine, Alkohol, Wasser, Salze, Stabilisatoren, Farbstoffe, Parfüme, etherische Öle.

Eine erfindungsgemäße Zubereitung wird durch Einarbeitung eines α-CD/Vitamin F Komplexes in eine übliche kosmetische oder dermatologische Formulierung hergestellt. Vorzugsweise erfolgt die Herstellung durch Dispergieren des Komplexes in Wasser sowie einmischen der wässrigen Dispersion in den lipophilen Teil der Emulsion. Dieser Verfahrensschritt ist essentiell für die kosmetische Qualität der Emulsion. Hautpflegeprodukte zeichnen sich beim Auftragen auf die Haut durch ein angenehmes Hautgefühl aus. Setzt man die Komplexe ohne vorherige Dispergierung in kosmetische Emulsionen ein, ergibt sich beim Auftragen ein sandiges bis kratziges Hautgefühl. Wird der Komplex nicht in der beschriebenen Weise durch Dispergieren zu Teilchengrössen<200Micrometer aufbereitet ist ein zusätzlicher Arbeitsschritt wie mahlen bzw. sieben zwingend. Durch die Dispergierung können Komplexe beliebiger Teilchengröße, also auch Granulate, ohne zusätzliche Zerkleinerung großer Komplexteilchen in kosmetische Formulierungen wie Emulsionen eingesetzt werden. Die Dispergierung kann über einen Zeitraum von 10 Minuten bis 2 Stunden bei Temperatur von 20°C bis 80°C, bevorzugt 30 Minuten bei 30-50°C, erfolgen.

Die folgenden Beispiele dienen der weiteren Erläuterung der Erfindung.

### Beispiel 1: Komplexierung von Linolsäure mit α-Cyclodextrin

a.) 0,1 mol α-Cyclodextrin wurden mit 100g H₂O versetzt, 0,1mol Linoläure zugegeben, homogenisiert und 30h bei RT sowie 8h bei 70°C gerührt. Die Wirkstoffkombination wird in Wasser dispergiert, filtriert, mit Wasser gewaschen und bei 40°C unter Vakuum getrocknet.
b.) 0,1 mol α-Cyclodextrin wurden mit 100g H₂O versetzt, 0,05mol Linoläure zugegeben, homogenisiert und 30h bei RT sowie 8h bei 70°C gerührt. Die Wirkstoffkombination wird in Wasser dispergiert, filtriert, mit Wasser gewaschen und bei 40°C unter Vakuum getrocknet.
c.) 0,1 mol α-Cyclodextrin wurden mit 100g H₂O versetzt, 0,033 mol Linoläure zugegeben, homogenisiert und 70h bei RT sowie 8h bei 70°C gerührt. Die Wirkstoffkombination wird in Wasser dispergiert, filtriert, mit Wasser gewaschen und bei 40°C unter Vakuum getrocknet.
d.) 0,1 mol α-Cyclodextrin wurden mit 100g H₂O versetzt, 0,025 mol Linoläure zugegeben, homogenisiert und 70h bei RT sowie 8h bei 70°C gerührt. Die Wirkstoffkombination wird in Wasser dispergiert, filtriert, mit Wasser gewaschen und bei 40°C unter Vakuum getrocknet.

### Beispiel 2: Herstellung der physikalischen Mischung α-Cyclodextrin/Linolsäure 4:1 (Vergleichsbeispiel)

α-Cyclodextrin 0,01 mol wurden in eine Reibschale eingewogen und mit 0,0025 mol Linolsäure intensiv verrieben, bis ein homogenes Pulver erhalten wurde.

### Beispiel 3: Bestimmung der Lagerstabilität von Komplexen aus α-Cyclodextrinen und Linolsäure sowie als physikalische Mischung, entsprechend einem 4:1-Komplex von α-Cyclodextrin mit Linolsäure

bei 45°C gelagert
Komplex aus α-Cyclodextrinen und Linolsäure 1:1
Komplex aus α-Cyclodextrinen und Linolsäure 2:1
Komplex aus α-Cyclodextrinen und Linolsäure 3:1
Komplex aus α-Cyclodextrinen und Linolsäure 4 : 1
Mischung der Wirkstoffkombination aus α-Cyclodextrinen und Linolsäure der molaren Verhältnisse von 1:1, 2:1, 3:1 und 4:1. Physikalische Mischung von α-Cyclodextrin 4:1 Linolsäure.
Alle Ansätze werden in offenen Schalen bei 45°C im Trockenschrank gelagert.
Der Gehalt an Linolsäure der physikalischen Mischung und der Wirkstoffkombinationen wurde über NMR bestimmt.

Fig: 1 zeigt den Linolsäure-Gehalt in den gelagerten Proben in Abhängigkeit von der Lagerdauer.

### Beispiel 4: Herstellung kosmetischer Formulierungen mit einer Wirkstoffkombination von α-Cyclodextrin mit Linolsäure.

### a) Herstellung einer Sonnenschutzcreme mit einer Wirkstoffkombination von α-Cyclodextrin mit Linolsäure.

Komponenten A mischen und auf 60°C erhitzen, B zu A zugeben, homogenisieren, Komponente C 15min. bei 60°C dispergieren und in AB einmischen.

Zusammensetzung und Gewichtsanteile:

| Wirkstoffe | Gewichtsanteile |
|---|---|
| A | |
| Octyl Palmitat | 2,5% |
| Octylstearat | 3,5% |
| Polyglycerol-2 Sesquiisostearat | 2,0% |
| Cyclomethicone, Dimethiconol | 3,0% |
| Lauryl Dimethicon | 2,0% |
| Octyl Dimethicone Ethoxy Glycosid, Cyclomethicon | 12,0% |

| B | |
|---|---|
| Titan Dioxid | 5,0% |
| Polymethylsilsesquioxan | 1,0% |
| Zink Oxyde | 2,0% |

| C | |
|---|---|
| Glycerin | 2,0% |
| Methylparaben | 0,1% |
| Natriumchlorid | 0,4% |
| Wasser | 59,0% |
| γ-Cyclodextrin-α-Tocopherol-Komplex | 1,5% |
| α-Cyclodextrin-Linolsäure Komplex | 4,0% |

### b) Herstellung einer W/O Bodylotion mit einer Wirkstoffkombination von α-Cyclodextrin mit Linolsäure

Komponenten A auf 70°C erwärmen, B nach 30 min. dispergieren bei Raumtemperatur, auf 70°C erwärmen und in A einarbeiten, gut homogenisieren, unter Rühren auf 40°C abkühlen, C zugeben, bis Erkalten weiter rühren.

Zusammensetzung und Gewichtsanteile:

| Wirkstoffe | Gewichtsanteile |
|---|---|
| A | |
| Bienenwachs | 3,0% |
| Hostacerin | 3,0% |
| BELSIL® CM 1000 | 5,0% |
| BELSIL® DM 1plus | 7,0% |
| BELSIL® PDM 20 | 4,55% |
| BELSIL® SPG 128 VP | 12,0% |

| B | |
|---|---|
| Natriumchlorid | 2,0% |
| Wasser | 59,4% |
| α-Cyclodextrin-Linolsäure Komplex | 4,0% |

| C | |
|---|---|
| Kathon | 0,05% |

### c) Herstellung einer After Sun Lotion mit einer Wirkstoffkombination von α-Cyclodextrin mit Linolsäure

Komponenten A und B auf 75°C erwärmen. Komponente D 30min. bei Raumtemperatur dispergieren, in B einarbeiten, A unter Rühren der Mischung der Komponenten B und D zufügen. Nach 5 min. C zugeben und auf 40°C abkühlen. E zugeben und unter Rühren auf Raumtemperatur abkühlen.

Zusammensetzung und Gewichtsanteile:

| Wirkstoffe | Gewichtsanteile |
|---|---|
| A | |
| Cetylalkohol | 1,5% |
| Mineralöl | 5,0% |
| Stearinsäure | 5,0% |

| B | |
|---|---|
| Allantoin | 0,5% |
| Propylenglykol | 3,0% |
| Wasser | 45,0% |

| C | |
|---|---|
| Cyclomethicone, Dimethicone | 15,0% |
| Phenyl Trimethicone | 2,0% |

| D | |
|---|---|
| α-Cyclodextrin-Linolsäure Komplex | 2,0% |
| γ-Cyclodexextrin-Retinol Komplex | 0,4% |
| Wasser | 20,0% |

| E | |
|---|---|
| Phenoxyethanol, Methylparaben, Butylparaben, Ethylparaben, Propylparaben, | 0,3% |
| Parfüm | 0,3% |

### d) Herstellung einer O/W Bodylotion mit einer Wirkstoffkombination von α-Cyclodextrin mit Linolsäure

Komponente A 30 min. bei Raumtemperatur dispergieren, auf 75°C erwärmen, B einarbeiten, C auf 70°C erwärmen und langsam in AB einrühren, dann D einrühren auf Raumtemperatur abkühlen.

Zusammensetzung und Gewichtsanteile:

| Wirkstoffe | Gewichtsanteile |
|---|---|
| A | |
| Carbopol | 0,1% |
| Wasser | 80,0% |
| Glycerin | 3,0% |
| α-Cyclodextrin-Linolsäure Komplex | 4,4% |

| B | |
|---|---|
| Triethanolamin | 0,9% |

| C | |
|---|---|
| Stearinsäure | 0,8% |
| Isopropylmyristat | 3,0% |
| Nexbase | 2,0% |
| Arlacel 165 | 1,5% |
| Cetylalkohol | 1,0% |

| D | |
|---|---|
| BELSIL® | 3,0% |
| BHT | 0,3% |

### e) Herstellung einer O/W Gesichtscreme mit einer Wirkstoffkombination von α-Cyclodextrin mit Linolsäure

Komponente A + B getrennt auf ca. 65°C erwärmen. Phase B ca. 10 min. dispergieren, vorlegen und Phase A unter Rühren zugeben, homogenisieren.

Zusammensetzung und Gewichtsanteile:

| Wirkstoffe | Gewichtsanteile |
|---|---|
| A | |
| Stearyl-glucoside | 3% |
| Glyceryl Stearate | 2% |
| Stearyl Alkohol | 1% |
| Decyl Cocoate | 10% |
| Cetearyl Ethylhexanoate | 9% |

| B | |
|---|---|
| Glycerin | 3% |
| Wasser | 62% |
| α-Cyclodextrin-Linolsäure Komplex | 9% |
| γ-Cyclodextrin-Retinol Komplex | 1% |

### f) Herstellung einer O/W Feuchtigkeitscreme mit einer Wirkstoffkombination von α-Cyclodextrin mit Linolsäure

Komponente A + B getrennt auf 65°C erwärmen, Phase B ca. 10 min. dispergieren, vorlegen und Phase A unter Rühren zugeben, dann Phase C. Die erhaltene Cremeformulierung homogenisieren.

Zusammensetzung und Gewichtsanteile:

| Wirkstoffe | Gewichtsanteile |
|---|---|
| A | |
| Stearyl-glucoside | 3,0% |
| Glyceryl Stearate | 2,1% |
| Stearyl Alkohol | 0,9% |
| Ethylhexyl-Stearate | 10,0% |
| Caprylic / Capric Triglyceride | 10,0% |
| Avocadoöl | 3,7% |

| B | |
|---|---|
| Glycerin | 3,0% |
| Wasser | 59,0% |
| α-Cyclodextrin-Linolsäure Komplex | 6,0% |
| | |

| C | |
|---|---|
| Sodium Lactate, Urea, Lactic Acid | 2,0% |
| Tocopherylacetat | 0,3% |

### g) Herstellung eines Flüssig Make-up mit Wirkstoffkombination von α-Cyclodextrin mit Linolsäure

A auf 75°C erwärmen, B unter Turrax in A einarbeiten, auf gute Pigmentverteilung achten, C 20 min. bei 50°C dispergieren, unter Turrax in AB einemulgieren, unter Rühren auf 40°C kühlen, D einrühren, auf RT abkühlen, kalt mit Turrax homogenisieren.

Zusammensetzung und Gewichtsanteile:

| Wirkstoffe | Gewichtsanteile |
|---|---|
| A | |
| Weißes Bienenwachs | 2,70% |
| Polyglyceryl-2 Sesquiisostearate | 2,40% |
| Dimethicone | 10,00% |
| Dimethicone | 2,50% |
| Octyl Dimethicone Ethoxy Glucosid, Cyclomethicone | 11,00% |
| Trimethylsiloxysilicat | 1,50% |

| B | |
|---|---|
| Eisenoxid | 1,45% |
| Talk | 5,00% |
| Titan Dioxide; | 7,00% |

| C | |
|---|---|
| Natriumchlorid | 2,00% |
| Wasser | 50,00% |
| γ-Cyclodextrin-α-Tocopherol-Komplex | 1,70% |
| α-Cyclodextrin-Linolsäure Komplex | 2,40% |

| D | |
|---|---|
| Methylchloroisothiazolinone | 0,05% |
| Parfüm | 0,30% |

### h) Herstellen einer Körperemulsion mit Wirkstoffkombination von α-Cyclodextrin mit Linolsäure

Die Rohstoffe A werden in einem Becherglas vorgelegt auf 65°C erwärmt, die Rohstoffe B in einem Rührgefäß bei 50°C 20 min. dispergiert. Beide Mischungen emulgiert man bei 65°C mit einem schnelllaufenden Flügelrührer und lässt unter weiterem Rühren auf 40°C abkühlen und homogenisiert mit dem Ultra-Turrax (max. 500 Upm). Die in der Creme gelöste Luft wird durch vorsichtiges Anlegen von Wasserstrahlvakuum entfernt.

| Wirkstoffe | Gewichtsanteile |
|---|---|
| A | |
| Glycerinmonomyristat | 1,4% |
| Stearinsäure | 1,2% |
| Cetylalkohol | 0,5% |
| Isopropylpalmitat | 5,0% |

| B | |
|---|---|
| Wasser dest. | 87,5% |
| Methylparaben | 1,0% |
| α-Cyclodextrin-Linolsäure Komplex | 3,4% |

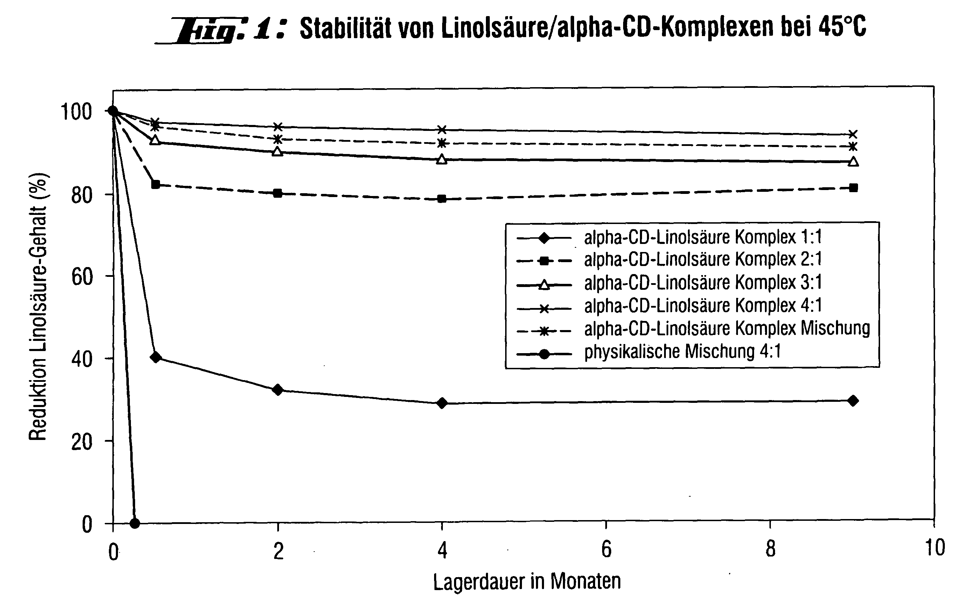

## Patentansprüche

1. Mischung aus Cyclodextrin und einer ungesättigten Fettsäure mit einer Kettenlänge größer bzw. gleich 18 Kohlenstoffatomen, die mindestens zwei Doppelbindungen aufweist, enthaltend
einen 4:1 Komplex von alpha-CD undder ungesättigten Fettsäure in einer Menge von 10 bis 90 Gew. %, oder
einen 3:1 Komplex von alpha-CD und der ungesättigten Fettsäure in einer Menge von 40 bis 80 Gew. %, oder
einen 2:1 Komplex von alpha-CD und der ungesättigten Fettsäure in einer Menge von 30 bis 70 Gew. %, oder
einen 1:1 Komplex von alpha-CD und der ungesättigten Fettsäure in einer Menge von 20 bis 60 Gew. %.

2. Zubereitung enthaltend eine Mischung gemäß Anspruch 1 **dadurch gekennzeichnet, dass** sie ferner eine oder mehrere der folgenden Komponenten enthält: lineare wie cyclische Silikonöle, feuchthaltende Mittel, Substanzen, welche die Haut pflegen, Gel-Bildner, O/W- oder W/O-Emulgatoren, Konservierungsmittel, Bakterizide, Antioxidantien, Sonnenschutzfilter, anorganische Pigmente, Selbstbräunungsmittel, Zusatzstoffe- und Hilfsstoffe, Konsistenzgeber, Füllstoffe, Elektrolyte, Vitamine, Alkohol, Wasser, Salze, Stabilisatoren, Farbstoffe, Parfüme, etherische Öle.

## Claims

1. Mixture of cyclodextrin and an unsaturated fatty acid with a chain length greater than or equal to 18 carbon atoms which has at least two double bonds, comprising
a 4:1 complex of alpha-CD and the unsaturated fatty acid in an amount of from 10 to 90% by weight, or
a 3:1 complex of alpha-CD and the unsaturated fatty acid in an amount of from 40 to 80% by weight, or
a 2:1 complex of alpha-CD and the unsaturated fatty acid in an amount of from 30 to 70% by weight, or
a 1:1 complex of alpha-CD and the unsaturated fatty acid in an amount of from 20 to 60% by weight.

2. Preparation comprising a mixture according to Claim 1, **characterized in that** it also comprises one or more of the following components: linear and cyclic silicone oils, humectant agents, substances which care for the skin, gel formers, O/W or W/O emulsifiers, preservatives, bactericides, antioxidants, sunscreen filters, inorganic pigments, self-tanning agents, additives and auxiliaries, consistency-imparting agents, fillers, electrolytes, vitamins, alcohol, water, salts, stabilizers, dyes, perfumes, essential oils.

## Revendications

1. Mélange de cyclodextrine et d'un acide gras insaturé présentant une longueur de chaîne supérieure ou égale à 18 atomes de carbone, qui présente au moins deux doubles liaisons, contenant
un complexe 4:1 d'alpha-CD et de l'acide gras insaturé en une quantité de 10 à 90% en poids, ou
un complexe 3:1 d'alpha-CD et de l'acide gras insaturé en une quantité de 40 à 80% en poids, ou
un complexe 2:1 d'alpha-CD et de l'acide gras insaturé en une quantité de 30 à 70% en poids, ou
un complexe 1:1 d'alpha-CD et de l'acide gras insaturé en une quantité de 20 à 60% en poids.

2. Composition contenant un mélange selon la revendication 1, **caractérisée en ce qu'**elle contient en outre un ou plusieurs des composants suivants : les huiles de silicone linéaires et cycliques, les agents de rétention de l'humidité, les substances qui soignent la peau, les agents de formation d'un gel, les émulsifiants H/E ou E/H, les conservateurs, les bactéricides, les anti-oxydants, les filtres de protection solaire, les pigments inorganiques, les auto-bronzants, les additifs et auxiliaires, les agents conférant une consistance, les charges, les électrolytes, les vitamines, les alcools, l'eau, les sels, les stabilisateurs, les colorants, les parfums, les huiles essentielles.
